# EUROPEAN PATENT APPLICATION

(11) **EP 1 604 609 A1**
(43) Date of publication of application: **14.12.2005**
(21) Application number: 04711459.0
(22) Date of filing: 16.02.2004
(51) Int. Cl.: A61B 5/04, A61B 5/05, A61B 5/145, A61N 1/04, A61N 5/06, G01N 27/30, G01N 21/64, G01J 5/10

(54) **LINEAR DEVICE**

(30) Priority: 17.02.2003 JP 2003038609
(71) Applicant: Toyo Precision Parts MFG. Co. Ltd., Kashihara-shi, Nara 634-0836 (JP); Yasuzawa, Mikito, Tokushima-shi, Tokushima 770-0865 (JP)
(72) Inventor: YASUZAWA, Mikito, Tokushima-shi, Tokushima 770-0865 (JP); IMAI, Shinji, Kashiba-shi, Nara 639-0222 (JP)
(74) Representative: Wagner, Karl H.
(86) International application number: PCT/JP2004/001669
(87) International publication number: WO 2004/071294

(57) **Abstract**

The object of the present invention is to provide a linear device which is very thin, can be inserted into a desired region of a subject, and minimizes the damage to the subject.

The linear device is a linear member, which comprises a base layer extending in the axial directions of the linear member and a plurality of layers formed on the base layer and extending in the axial directions of the linear member. Two or more of said plurality of layers are conductive layers and two or more of said plurality of layers are insulating layers. Each insulating layer is disposed between the conductive layers. When the linear device is embedded in or its front end is inserted into a region of a subject, electric stimuli can be given to the region and the electric resistance of the region can be measured. Because the linear device is very thin, pain or uncomfortable feeling at the region is slight when it is inserted or embedded in a region of the human body or the like.

## Description

### Technical Field

This invention relates to a linear device. More specifically, this invention relates to a linear device which is applied to or embedded in various regions of organisms or the like, give electric, thermal, or optical stimuli to the regions, and detect and measure the changes occurring in the regions electrically, electrochemically, or optically.

### Background Art

Enzyme sensors with enzyme-applied electrodes have been used to date to measure the density of sugar, amino acid, etc. in organisms. Enzymes oxidize and deoxidize sugar, amino acid, etc. selectively to generate molecules and ions, and enzyme sensors detect the quantity of such molecules and ions as the values of currents passing through their electrodes to determine the density of sugar, amino acid, etc.

A glucose sensor is disclosed in the Japanese Unexamined Patent Publication No. 1993-60722 (hereinafter "Prior Art 1"). Another glucose sensor is disclosed in the literature "A new amperometric glucose microsensor: in vitro and short-term in vivo evaluation" by W. Kenneth Ward, Lawrence B. Jansen, Ellen Anderson, Gerard Reach, Jean-Claude Klein, and George S. Wilson (Biosensors & Bioelectronics 17, 2002, pp. 181-189) (hereinafter "Prior Art 2").

The glucose sensor of Prior Art 1 comprises a rod-like titanium electrode, an insulating layer which is a glass tube housing the titanium electrode, and a silver-plate electrode made of a silver plate wrapped around the insulating layer. The surface of the titanium electrode is oxidized to be a titanium-oxide layer, and glucose oxidase is applied to the surface of the titanium-oxide layer.

When the glucose sensor is inserted into the human body or the tip of the glucose sensor is dipped into the blood in a blood vessel and voltage is applied between the titanium and silver-plate electrodes, a current corresponding to the density of glucose passes through the titanium and silver-plate electrodes. Thus, the density of glucose in the tissues and blood of organisms can be measured.

The glucose sensor of Prior Art 2 comprises a rod-like platinum-iridium-alloy electrode, an insulating layer which is a Teflon tube housing the Pt-Ir -alloy electrode, and a silver-wire electrode made of a silver wire wrapped around the insulating layer. Protein is caused to stick fast to the surface of the Pt-Ir-alloy electrode and glucose oxidase is bonded to the protein with the crosslinking agent of glutaraldehyde so as to measure the density of glucose in the tissues and blood of organisms.

However, the diameter of the glucose sensor of Prior Art 1 is about 0.8 mm and that of the glucose sensor of Prior Art 2 is at least 0.35 mm; accordingly, when the glucose sensors are stuck into the human body, many cells are damaged. If the glucose sensors are embedded and left in a region of the human body, the region aches or feels uncomfortable.

If the diameter of such a glucose sensor is reduced, the number of cells to be damaged and the pain or uncomfortable feeling in such a region are reduced. However, the strength of the glucose sensor too is reduced; accordingly, it may be difficult to insert the glucose sensor into the human body or the glucose sensor may buckle while it is being inserted into the human body. Thus, it may be difficult to apply the glucose sensor to a desired region of the human body. If the glucose sensor is bent and broken in a region of the human body, the sensor stops functioning and the region may be damaged and ache.

Besides, because the glucose sensor of Prior Art 1 has a large diameter and its titanium-oxide electrode too is large, it cannot be used for the measurement of the density of glucose in minute regions such as local regions in the brain.

### Disclosure of Invention

Accordingly, the object of the present invention is to provide a linear device which is slim, can be inserted into a desired region of a subject, and minimizes the damage to the subject.

According to the first feature of the present invention, there is provided a linear device which is a linear member comprising (i) a base layer extending in the axial directions of the linear member and (ii) a plurality of layers formed on the base layer and extending in the axial directions of the linear member. One of said plurality of layers is a conductive layer and one of said plurality of layers is an insulating layer.

The advantages offered by the first feature of the present invention are as follows. When two linear devices are disposed side by side in a substance and voltage is applied to the conductive layers of the linear devices, a current passes through the substance between the linear devices. Thus, an electric stimulus is given to the substance. Besides, because the current value is dependent on the properties of the substance, the substance can be identified and its density can be determined.

According to the second feature of the present invention, there is provided the linear device according to the first feature, wherein the front end of the linear member is pointed.

The advantages offered by the second feature of the present invention are as follows. Because the front end of the linear member is pointed, the resistance when the linear device is inserted into a subject such as an organism is small. Besides, because the contacting area between the conductive layer and the subject is small, a current can be caused to pass through a minute region and therefore a substance in a minute region can be detected and measured.

According to the third feature of the present invention, there is provided the linear device according to the first or second feature, wherein (i) the conductive layer is formed on one side of the base layer, (ii) the insulating layer is formed so as to cover the surface of the conductive layer, and (iii) the conductive layer is exposed at the front end of the linear member to constitute a contacting part.

The advantage offered by the third feature of the present invention is as follows. The contacting area between the conductive layer and a subject can be adjusted by changing the size of the contacting part.

According to the fourth feature of the present invention, there is provided the linear device according to the third feature, wherein a platinum layer is formed on the contacting part.

The advantage offered by the fourth feature of the present invention is as follows. Brought into direct contact with a subject such as an organism is not the conductive layer, but the layer of chemically stable platinum. Thus, the conductive layer is effectively prevented from coming into direct contact with the subject and, hence, from affecting the subject directly; therefore, the conductive layer can be made of various materials.

According to the fifth feature of the present invention, there is provided the linear device according to the first, second, third, or fourth feature, wherein two or more of said plurality of layers are conductive layers and two or more of said plurality of layers are insulating layers. Each insulating layer is disposed between the conductive layers.

The advantages offered by the fifth feature of the present invention are as follows. When voltage is applied between conductive layers at the rear end of the linear member, a potential difference occurs between the conductive layers at the front end of the linear member. Accordingly, if the linear device is inserted or embedded in a region of a subject such as a human body, electric stimuli can be given to the region and the electric resistance of the region can be measured. Besides, because the linear device is slim, pain and uncomfortable feeling is minimized when it is inserted or embedded in the human body or the like.

According to the sixth feature of the present invention, there is provided the linear device according to the first, second, third, fourth, or fifth feature, wherein one of said plurality of layers is of a superelastic alloy.

The advantages offered by the sixth feature of the present invention are as follows. Because the linear device is highly elastic, the linear device does not easily buckle or break when it is inserted into an organism or the like; accordingly, the linear device can be inserted into a desired region of a subject without fail and damage to the region can be minimized.

According to the seventh feature of the present invention, there is provided the linear device according to the first, second, third, fourth, or fifth feature, wherein one of said plurality of layers is of a superelastic resin.

The advantages offered by the seventh feature of the present invention are as follows. Because the linear device is highly elastic, the linear device does not easily buckle or break when it is inserted into an organism or the like; accordingly, the linear device can be inserted into a desired region of a subject without fail and damage to the region can be minimized.

According to the eighth feature of the present invention, there is provided the linear device according to the first, second, third, fourth, fifth, sixth, or seventh feature, wherein one of said plurality of layers is of a shape-memory material.

The advantages offered by the eighth feature of the present invention are as follows. Because one of said plurality of layers is of a shape-memory material, the linear device assumes a certain shape in a subject if a right shape-memory material is chosen. Accordingly, the front end of the linear device can be positioned exactly at a desired region of the subject. Thus, the linear device is capable of giving electric stimuli or the like to a desired region accurately and measuring the electric resistance or the like of a desired region of the subject accurately.

According to the ninth feature of the present invention, there is provided the linear device according to the first, second, third, fourth, fifth, sixth, seventh, or eighth feature, wherein the width of the linear member is 1-200 µm.

The advantages offered by the ninth feature of the present invention are as follows. Because the linear member is very thin, the region to which the linear device has been applied to is less affected by the linear device. When it is applied to a region of the human body, pain or uncomfortable feeling is slight.

According to the tenth feature of the present invention, there is provided the linear device according to the first, second, third, fourth, fifth, sixth, seventh, eighth, or ninth feature, wherein the linear member has an axial core serving as the base layer.

The advantages offered by the tenth feature of the present invention are as follows. Because the linear member has an axial core, the strength of the linear member is high. When the linear device is stuck into a subject, it does not easily buckle or break. Accordingly, the linear device can be stuck into a subject to locate its front end at a desired region of the subject without fail. Besides, the region is less affected by the linear device. Moreover, said plurality of layers are formed concentrically on the axial core. Namely, each layer is symmetrical with respect to the longitudinal center axis of the linear member. Accordingly, when the linear device is turned and inserted into a subject, the state of contact between the linear device and the subject is prevented from being affected by the turning of the linear device. Therefore, regardless of the turning angle of the linear device, an electric stimulus can be applied to an exact point and electric resistance at an exact point can be measured. Furthermore, with high reproducibility, an electric stimulus can repeatedly be applied to an exact point of a subject and the resistance at an exact point of a subject can repeatedly be measured.

According to the eleventh feature of the present invention, there is provided the linear device according to the first, second, third, fourth, fifth, sixth, seventh, eighth, ninth, or tenth feature, wherein a detecting agent, which reacts on a certain substance to produce another one, is applied to the surface of one of the conductive layers at the front end of the linear member.

The advantage offered by the eleventh feature of the present invention is as follows. When voltage is applied between the conductive layer with the detecting agent and another conductive layer, the detecting agent reacts with a certain substance, if any near the front end of the linear device, to produce another substance. The production of said another substance may reduce oxygen. The current and the potential difference between the two conductive layers change in accordance with the quantity and the production rate of the produced substance or the quantity of reduction of oxygen and its reduction rate. Accordingly, the presence or absence and quantity or density of said certain substance can be detected and measured by measuring the changes of the current and the potential difference between the two conductive layers.

According to the twelfth feature of the present invention, there is provided the linear device according to the first, second, third, fourth, fifth, sixth, seventh, eighth, ninth, tenth, or twelfth feature, wherein one side of the front end of the linear member is provided with a treating region which includes conductive surfaces and insulating surfaces arranged alternately in the directions of the longitudinal center axis of the linear member, each conductive surface being part of the outer surface of one of the conductive layers and each insulating surface being part of the outer surface of one of the insulating layers.

The advantage offered by the twelfth feature of the present invention is as follows. Because conductive surfaces and insulating surfaces are to be formed on one side of the front end of the linear member, conductive and insulating surfaces of any desired length can be formed regardless of the thickness of the conductive and insulating layers. Therefore, a treating region most suitable for each use can be formed.

According to the thirteenth feature of the present invention, there is provided the linear device according to the twelfth feature, wherein the front end of the linear member is provided with a protector of an insulating material to cover the front end.

The advantages offered by the thirteenth feature of the present invention are as follows. Because the front end of the linear member is covered by the protector, the front end is prevented from being damaged when the linear device is inserted into a subject. Especially if the protector is given a cone-like, tapering-off shape, the resistance when the linear device is inserted into a subject is reduced. Thus, the damage to the subject and the linear device is reduced. Moreover, because the protector is made of an insulating material, electricity does not flow through the front end of the linear member when electricity is allowed to flow through conductive layers. Namely, electricity does not flow between the front ends of the voltage-applied conductive layers. Accordingly, linear devices do not vary in sensitivity and precision if the shapes of their front ends vary due to manufacturing errors.

### Brief Description of Drawings

Fig. 1 is a schematic illustration of a typical linear device of the present invention. Fig. 1 (A) is a side view of the linear device; Fig. 1 (B), an enlarged view of the treating region 10 of the linear device.
Fig. 2 (A) is a schematic side view of the linear device of Fig. 1 before the treating region 10 is formed at its front end; Fig. 2 (B), an end view taken along the arrowed line B-B of Fig. 2 (A).
Fig. 3 is a schematic illustration of another embodiment of linear device in accordance with the present invention. Fig. 3 (A) is a side view of the linear device; Fig. 3 (B), an enlarged view of the treating region 10 of the linear device.
Fig. 4 is a schematic illustration of a device to form layers on a rod 100 which later becomes an axial core 2.
Fig. 5 (A) is an illustration of another mechanism for rotating a rod 100. Fig. 5 (B) is an end view taken along the arrowed line B-B of Fig. 5 (A). Fig. 5 (C) is an illustration of a mechanism for rotating a plurality of rods 100.
Fig. 6 is a schematic illustration of a device which has a holder 60 for holding a plurality of rods 100 and forms layers on a plurality rods 100.
Fig. 7 (A) is a plan view taken along the arrowed line VI-VI of Fig. 6. Fig. 7 (B) is a side view taken along the arrow B of Fig. 7 (A). Fig. 7 (C) is an illustration of another mechanism to rotate a plurality of rods 100.
Fig. 8 is a schematic illustration of still another embodiment of linear device in accordance with the present invention. Fig. 8 (A) is a side view of the linear device. Fig. 8 (B) is a sectional view taken along the arrowed line B-B of Fig. 8 (A). Fig. 8 (C) is a sectional view take along the arrowed line C-C of Fig. 8 (A).

### Best Mode for carrying out the Invention

A preferred embodiment of the present invention will be described below by referring to the drawings.

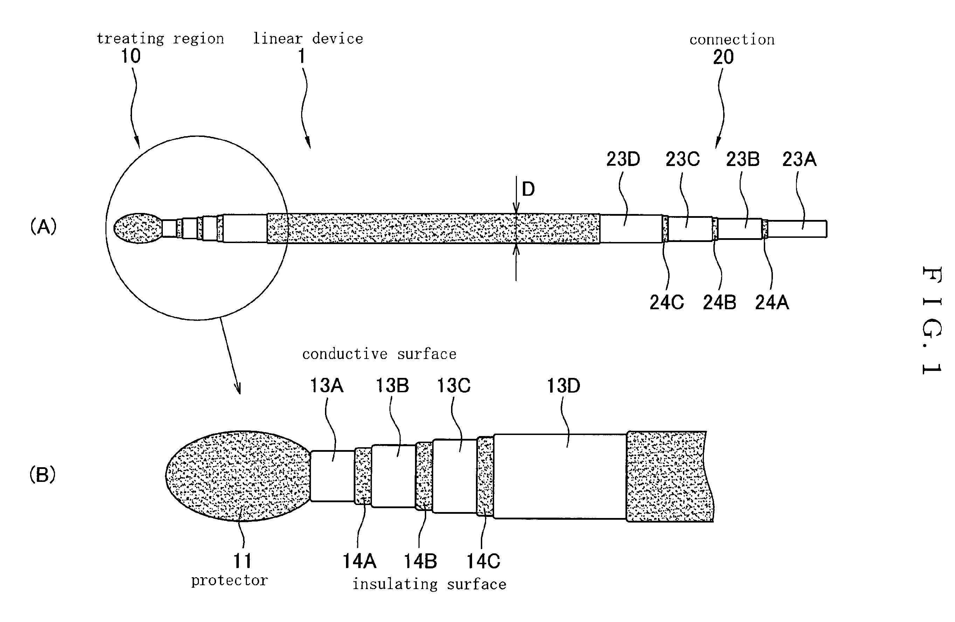

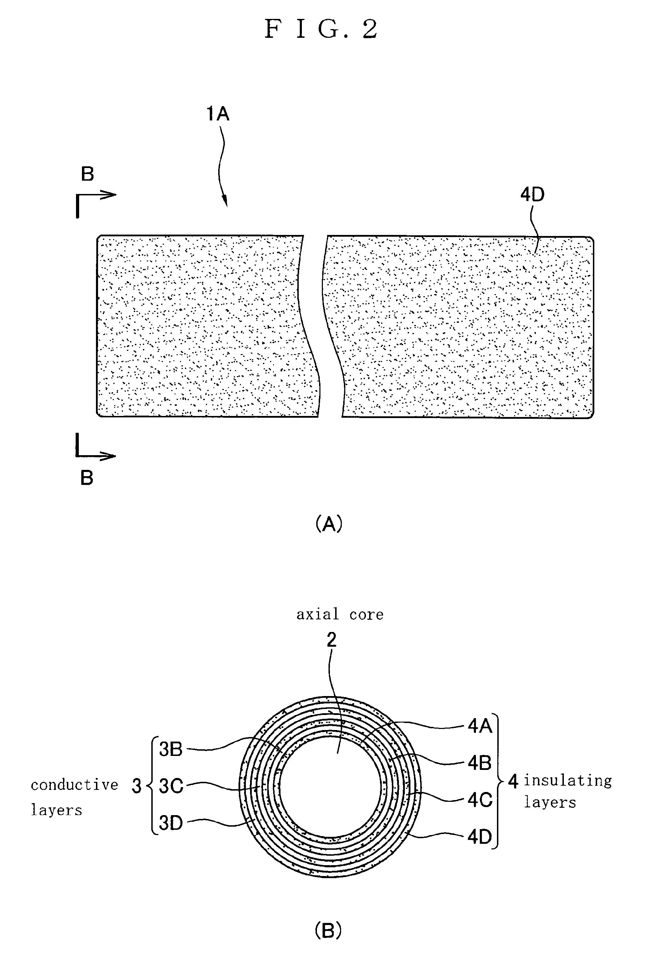

Fig. 1 is a schematic illustration of a linear device 1 with a treating region 10 in accordance with the present invention. Fig. 1 (A) is a side view of the linear device 1; Fig. 1 (B), an enlarged view of the treating region 10. Fig. 2 (A) is a side view of a linear device 1A without a treating region 10; Fig. 2 (B), an end view taken along the arrowed line B-B of Fig. 2 (A). As shown in Figs. 1 and 2, the linear device 1 is a linear member comprising a plurality of layers of an insulating material and a plurality of layers of a conductive material.

As shown in Fig. 2 (B), the linear device 1 further comprises a rod-like member 2 (hereinafter referred to as "axial core"). The axial core 2 is of a conducting material and has a circular cross section.

A plurality of thin layers are formed on the axial core 2. The layers are insulating layers 4A-D of an insulating material and conductive layers 3B-D of a conductive material, the former layers and the latter ones arranged alternately. More specifically, the insulating layer 4A lies between the axial core 2 and the conductive layer 3B; the insulating layer 4B, between the conductive layers 3B and 3C; the insulating layer 4C, between conductive layers 3C and 3D; and the insulating layer 4D lies on the conductive layer 3D.

The conductive layers 3B-D and the insulating layers 4A-D are formed by the thin-film method or the like such as the vapor-depositing method or the sputtering method or, alternatively, may be formed by any other methods. If the conductive layer is to be formed out of platinum, it can be fixed, or anchored, firmly by first forming an underlying layer out of another material and then forming the conductive layer out of platinum on the underlying layer.

Accordingly, when voltage is applied between the axial core 2 and the conductive layer 3B, between the conductive layers 3B and 3C, or between the conductive layers 3C and 3D at one end of the linear device 1A, a potential difference occurs between the voltage-applied conductors at the other end of the linear device 1A. When electrodes are connected to the axial core 2 and conductive layers 3B-D and voltage is applied between electrodes at the right end of the linear device 1A in Fig. 2 (A), a potential difference occurs between the voltage-applied conductors exposed at the left end (hereinafter referred to as "front end") of the linear device 1A.

Then, the front end of the linear device 1 is inserted or the linear device 1 is embedded in a subject such as a human body, and an electric stimulus is applied to the nerve or tissue of the subject in the vicinity of the front end of the linear device 1. Then, the resistance of the nerve or tissue can be measured by measuring the current passing from one of the voltage-applied two conductors to the other one.

The diameter "D" of the linear device 1 is 1-200 µm. Thus, the linear device 1 of the present invention is very thin as compared with the electrodes, glucose sensors, etc. of prior art. Accordingly, when the linear device 1 is inserted or embedded in a subject, the effects of the linear device 1 on the nearby tissue or matter are small. For example, when the linear device 1 is embedded in a subject, the space occupied by the linear device 1 is small. When the linear device 1 is stuck into an organism such as a human body, the damage to the nearby tissue is small. Thus, the damage to a subject which the linear device 1 is embedded in is held down and the pain or uncomfortable feeling of an organism such as a human body which the linear device 1 is embedded in is held down.

Part of a subject where an electric stimulus is given or part of a subject where an electric current passes (hereinafter referred to as "contact part") is determined by the areas of exposed parts of the axial core 2 and conductive layers 3B-D and the thicknesses of the insulating layers 4A-D. Because the diameter "D" of the linear device 1 is very small and the conductive layers 3B-D and the insulating layers 4A-D are thin, the contact part is very small.

Accordingly, an electric stimulus can be applied to very minute part of a subject and the resistance of very local part of a subject can be measured. Thus, bad effects due to electric stimuli applied to other parts than target part of subject can be prevented and the measuring accuracy of the linear device 1 is high.

If the linear device 1 is inserted in the brain of an organism, electric stimuli can be applied to certain cells or certain nerves. Accordingly, by observing the organism's responses to such stimuli, the functions of various parts of the brain can be ascertained.

Besides, by applying stimuli to a certain nerve or muscle, the growth of the nerve can be guided and controlled, or the muscle can be stimulated to recover its function, without affecting other nerves and muscles.

More specifically, by using the linear device 1, electric stimuli can be applied to a damaged nerve without applying electric stimuli to other normal nerves. Thus, without giving pain to the patient, the deterioration of the function of the damaged nerve can be held down. Because electric stimuli accelerate the growth of nerve cells, the recovery of damaged nerves can be accelerated.

If brain waves are abnormal, the abnormal brain waves can be put under sedation and controlled by stimulating a certain part of the brain. The visual performance, or acuity, can be recovered and improved by giving electric stimuli to visual cells and nerves. By stimulating a certain nerve or muscle of a heart developing arrhythmia, cardiac failure, or cardiac arrest, its normal function can be recovered. Thrombi can be resolved by electric stimuli if the linear device 1 is inserted into a blood vessel.

Moreover, the linear device 1 can be used as a stimulating device or the like to transmit information in the form of electric signals to the nerves of each sensory organ. If a sensory organ of an organism, such as an eye or an ear, is to be reproduced artificially, information acquired by a CCD camera or a microphone has to be transmitted accurately to the nerves of the organism through a stimulating device, without damaging the nerves. Namely, information acquired by a sensor and converted into electric signals has to be transmitted accurately to the nerves of the sensory organ without damaging the nerves. If the linear device 1 is used as such a stimulating device, stimuli can be applied to very minute part of an organism; therefore, electric signals can be transmitted accurately to a certain nerve without damaging it.

If a detecting agent, which reacts on a certain substance to produce another one, is applied to the exposed surface of one of the conductive layers 3B-D at the front end of the linear device 1, the presence or absence, quantity, and concentration of said certain substance can be detected and measured.

If glucose oxidase is applied to the exposed surface of the conductive layer 3B and glucose exists in the vicinity of the front end of the linear device 1, the glucose oxidase reacts on the glucose to produce hydrogen peroxide in accordance with the quantity of the glucose. Then, voltage is applied between the axial core 2 and the conductive layer 3B and the hydrogen peroxide is deoxidized at the conductive layer 3B; accordingly, the current between the axial core 2 and the conductive layer 3B changes in accordance with the quantity of hydrogen peroxide. Thus, because the current between the axial core 2 and the conductive layer 3B changes in accordance with the quantity of glucose in the vicinity of the front end of the linear device 1, the presence or absence, quantity, and concentration of glucose can be detected and measured.

In addition to enzymes such as glucose oxidase mentioned above, antigens, antibodies, polypeptides, receptors, acceptors, nucleic acid, sugar, cells, microbes, permselective membranes, nonspecific absorption-preventive membranes, chelating agents, crown ether, cyclodextrin, etc. may be used as the detecting agent.

The changes of potential difference instead of the above-mentioned currents between the axial core 2 and the conductive layers 3B-D may be measured. Optimum physical quantities may be chosen in accordance with detecting agents and produced-by-reaction substances.

Besides, if different detecting agents are applied to the different conductive layers 3B-D, a plurality of different substances can be detected and measured.

Moreover, the exposed surfaces of the conductive layers 3B-D may be coated with electroluminescence materials (hereinafter referred to as "EL materials"), which emit light of certain wavelengths when voltage is applied, such as polysilane, carbasole derivatives, and metal complexes. In this case, when voltage is applied between the axial core 2 and one of the conductive layers 3B-D, light of a certain wavelength is emitted from the front end of the linear device 1, stimulating the nerves or tissue in the vicinity of the front end optically. If EL materials emitting light of near-ultraviolet or ultraviolet wavelengths, such as polysilane, are used, ultraviolet light can be applied to cells, which affect the health of organisms, such as cancer cells and tumors, to kill them. If the exposed surface of one of the conductive layers 3B-D is coated with an EL material and an optical catalytic agent such as titanium oxide, cancer cells and the like can be killed effectively. If an EL material emitting infrared light is used, a subject can be not only stimulated optically but also heated.

Furthermore, the exposed surfaces of the conductive layers 3B-D may be coated with an electric-resistance heating material such as titanium-nickel alloy, platinum, silicon carbide, or carbon so as to connect the axial core 2 and the conductive layer 3B, or the conductive layers 3B and 3C, or the conductive layers 3C and 3D. In this case, when voltage is applied to the electric-resistance heating material, the electric-resistance heating material generates heat; accordingly, matter in the vicinity of the front end of the linear device 1 can be stimulated thermally. Thus, cancer cells and the like can be killed by heating without affecting cells and the like around the cancer cells and the like.

If a pharmacologically active agent is so applied to the exposed surfaces of the conductive layers 3B-D that the agent will be released electrically, thermally, or optically, the agent can be administered with a pinpoint accuracy by inserting the treating region 10 of the linear device 1 in certain minute part of cancer cells or the like. Poly methyl methacrylate, for example, contracts at pH 3 or so and expands over pH 6. If a pharmacologically active agent is wrapped in film of poly methyl methacrylate and attached to the exposed surfaces of the conductive layers 3B-D, the film of poly methyl methacrylate is unfolded to release the agent when voltage is applied between the axial core 2 and the conductive layers 3B-D to generate electrolytic bases and raise the pH in the vicinity of the conductive layers 3B-D to 6. Thus, a pharmacologically active agent can be administered to a desired place at a desired time by well-timed application of voltage between the axial core 2 and the conductive layers 3B-D.

The conductive layers 3B-D and the insulating layers 4A-D are formed concentrically on the axial core 2. Namely, each layer is symmetrical with respect to the longitudinal center axis of the linear device 1. Accordingly, when the linear device 1 is turned and inserted in a subject, the state of contact between the contact part of the linear device 1 and the subject can be prevented from being affected by the turning of the linear device 1. Therefore, regardless of the turning angle of the linear device 1, an electric stimulus can be applied to an exact point and electric resistance at an exact point can be measured. Besides, with high reproducibility, an electric stimulus can repeatedly be applied to an exact point of a subject and the resistance at an exact point of a subject can repeatedly be measured.

As shown in Fig. 2, the linear device 1 has the axial core 2 and, therefore, is strong. Accordingly, when it is stuck into a subject, it does not easily buckle or break under axial force.

Accordingly, the linear device 1 can be stuck into a subject to locate its front end at a desired part of the subject without fail.

If the axial core 2 is made of superelastic alloy, large torque can be transmitted. Accordingly, if the linear device 1 is turned and inserted in a subject with a relatively hard surface, the linear device 1 does not easily buckle or break. Thus, the linear device 1 can be inserted into the subject without fail.

Besides, if the axial core 2 is made of superelastic alloy, the elasticity of the linear device 1 is very high. Accordingly, if the linear device 1 is embedded in a subject and the subject is bent or deformed, the linear device 1 is not easily broken.

The axial core 2 may be made of an insulating material instead of a conductive material. If the axial core 2 is made of superelastic resin, the elasticity of the linear device 1 is very high. Accordingly, if the linear device 1 is embedded in a subject and the subject is heavily bent or deformed, the linear device 1 is not easily broken.

Moreover, the axial core 2 may be made of light-transmitting glass or resin such as optical fibers. In this case, when the front end of the linear device 1 is inserted into a subject and light is let through the axial core 2, light radiates from the front end of the linear device 1; accordingly, an optical stimulus can be given to a desired part of a subject in the vicinity of the front end of the linear device 1 and the characteristics of the desired part can be checked optically.

A laser beam can be let through the axial core 2 made of optical fibers to apply the laser beam to a desired part of a subject; accordingly, if the linear device 1 is used for the less-invasive laser treatment of cancer, herniation of a loose disc, etc., a laser beam can be applied to an exactly desired part of a subject. If two linear devices 1 are used to apply light to a desired part of a subject with one linear device 1 and receive the light reflected from the desired part with the other one, the change of temperature or pressure at the desired part can be measured based on the change of intensity of reflected light.

If a linear device 1 is provided with an axial core 2 capable of applying light to a desired part of a subject and, at the same time, receiving the light reflected from the desired part, the single linear device 1 is capable of measuring the change of temperature or pressure at the desired part of the subject.

Furthermore, if the exposed surfaces of the conductive layers 3B-D are coated with an EL material and voltage is applied between the axial core 2 and the conductive layer 3B, or between the conductive layers 3B and 3C, or between the conductive layers 3C and 3D, the EL material generates light of a certain wavelength. If there is in the subject a fluorescent material or the like which is excited by the light from the EL material and emits light, the light from the EL material can be detected with the axial core 2. Thus, the presence and the quantity of the fluorescent material can be detected and measured.

If a laser beam is let into the axial core 2 through its rear end and out of it through its front end while the linear device 1 is being inserted in a subject, the tissue of the subject in front of the front end of the linear device 1 is burned and a hole is made in the subject; accordingly, the linear device 1 can easily be inserted in the subject without turning the linear device 1 about its longitudinal center axis. In this case, the linear device 1 is inserted into the hole made in the subject by the laser beam; therefore, the linear device 1 is not exposed to the pushing force which it would be exposed to if there were no hole. Thus, the linear device 1 is prevented from being broken.

Not only the axial core 2 but also the conductive layers 3B-D may be made of superelastic alloy. In this case, the linear device 1 does not easily buckle or break when it is turned about its longitudinal center axis and inserted in a subject. Thus, the linear device 1 can be inserted in subjects without fail.

The conductive layers 3B-D may be made of superelastic alloy, while the axial core 2 may be made of other materials. In this case too, the linear device 1 does not easily buckle or break; accordingly, it can be inserted in subjects without fail.

The above superelastic alloy may be titanium-nickel (Ti-Ni) alloy, indium-thallium (In-Tl) alloy, copper-zinc (Cu-Zn) alloy, copper-zinc-X [Cu-Zn-X (Si, Sn, Al, or Ga)] alloy, copper-aluminum-nickel (Cu-Al-Ni) alloy, copper-gold-zinc (Cu-Au-Zn) alloy, copper-tin (Cu-Sn) alloy, nickel-aluminum (Ni-Al) alloy, iron-platinum (Fe-Pt) alloy, indium-cadmium (In-Cd) alloy, manganese-copper (Mn-Cu) alloy, silver-cadmium (Ag-Cd) alloy, gold-cadmium (Au-Cd) alloy, iron-palladium (Fe-Pd) alloy, iron-nickel-cobalt-titanium (Fe-Ni-Co-Ti) alloy, iron-nickel-carbon (Fe-Ni-C) alloy, iron-manganese-silicon (Fe-Mn-Si) alloy, titanium-aluminum-tin-zirconium-molybdenum (Ti-Al-Sn-Zr-Mo) alloy, titanium-aluminum-vanadium (Ti-Al-V) alloy, titanium-molybdenum-aluminum (Ti-Mo-Al) alloy, titanium-niobium (Ti-Nb) alloy, titanium-niobium-tin (Ti-Nb-Sn) alloy, titanium-vanadium-iron-aluminum (Ti-V-Fe-Al) alloy, or the like.

Alloys containing no copper, nickel, or cadmium harmful to organisms are suitable, particularly if the linear device 1 is to be embedded in organisms such as human bodies. As the quantity of nickel released from titanium-nickel alloy in the human body is smaller than the quantity of nickel released from stainless steel for the treatment of broken bones in the human body, the titanium-nickel alloy can be used in organisms.

In addition to the above superelastic alloys, the conductive layers 3B-D may be made of gold (Au), silver (Ag), copper (Cu), platinum (Pt), alloys such as platinum-iridium (Pt-Ir) alloy, palladium (Pd), nickel (Ni), titanium (Ti), carbon (C), polypyrrole, polythiophene, polyaniline, polyacethylene, etc.

If the insulating layers 4A-D are made of superelastic resin, the linear device 1 does not easily buckle or break when it is turned about its longitudinal center axis and inserted in a subject. Thus, the linear device 1 can be inserted in subjects without fail.

The insulating layers 4A-D may be made of superelastic resin, while the axial core 2 and the conductive layers 3B-D may be made of a material or materials other than superelastic alloy. In this case too, the linear device 1 can be inserted in subjects without fail.

The above superelastic resin may be polyisoprene, styrene-butadiene copolymer, polyethylene, fluororesin, polyethylene + nylon, polyethylene + perprene, polyester acrylate, polyester methacrylate, polysiloxane, silicon resin, polyvinyl chloride, chlorinated polyethylene, perprene, polyethylene + polyvinyl chloride, polyethylene + fluororesin, polyurethane, polyimide, polyamide, polysilane, or the like.

If the linear device 1 is to be embedded in organisms, it is desirable for the insulating layers 4A-D of the linear device 1 to be made of a superelastic resin, such as fluororesin or polysiloxane, which organisms are unlikely to reject, namely which are compatible with organisms.

In addition to the above superelastic resins, the insulating layers 4A-D may be made of PET (polyethylene terephthalate), polyphenylenediamine, polyurethane, nylon, polyvinyl chloride, polysiloxane, glass (SiO₂), polypropylene, polythiophene, polyester, polyethylene, urea resin, polysilane, polyaniline, metallic oxide, etc.

Besides, some of the above superelastic alloys, which the conductive layers 3B-D may be made of, are used as semiconductors. Accordingly, if the conductive layers 3B-D are made of a material of very high conductivity, the insulating layers 4A-D may be made of a superelastic alloy which can be used as semiconductors.

Moreover, the axial core 2, conductive layers 3B-D, and insulating layers 4A-D may be made of a so-called shape-memory material which gives the core and the layers certain shapes at the temperature of a subject. In this case, a certain shape is given to the linear device 1 without fail in a subject.

Accordingly, the linear device 1 can be given a shape convenient for storage and transport during storage and transport, while it is given a certain shape, for example a straight shape, at the time of its insertion into a subject and the certain shape is kept in the subject. Thus, the linear device 1 can easily be inserted into a subject. When it is inserted into a subject, it is prevented from buckling and breaking. Thus, the linear device 1 can be embedded in a desired place without fail. Besides, damage to the subject can be prevented. Moreover, as the linear device 1 is always in the above certain shape in the subject, the front end of the linear device 1 is prevented from getting out of position in the subject.

The linear device 1 may be made of a shape-memory material whose shape-changing temperature changes along the longitudinal center axis of the linear device 1. If (i) only the front end of the linear device 1 is made of a shape-memory material which gives the front end a certain shape at a temperature higher than the temperature of the subject and (ii) the front end is coated with such an electric-resistance heating material as was described earlier, only the front end can be put into a desired shape by applying voltage between the axial core 2 and the conductive layers 3B-D. If the front end of the linear device 1 is so designed that it will assume a spiral or curved shape at a certain temperature, the linear device 1 is prevented from moving in and coming off the subject without fail while the linear device 1 is giving stimuli to the subject.

Furthermore, if the front end of the linear device 1 is provided with a treating region 10 as shown in Fig. 1, electric stimuli can be applied to a subject more locally and minutely and substances in the vicinity of the front end of the linear device 1 can be detected more accurately.

The treating region 10 comprises conductive surfaces 13A-D and insulating surfaces 14A-D arranged alternately.

The conductive surfaces 13A-D are the exposed surfaces of the axial core 2 and the conductive layers 3B-D, respectively. The insulating surfaces 14A-D are the exposed surfaces of the insulating layers 4A-D. Thus, the treating region 10 is formed by exposing front-end part of the surface of the axial core 2 and front-end parts of the outer surfaces of the conductive layers 3B-D and insulating layers 4A-D.

Thus, the conductive surfaces 13A-D and insulating surfaces 14A-D of desired lengths, or areas, can be formed regardless of the thicknesses of the conductive layers 3B-D and insulating layers 4A-D.

Accordingly, the lengths of the conductive and insulating surfaces 13A-D and 14A-D can freely be adjusted, making them long or short as compared with the thicknesses of the conductive and insulating layers 3B-D and 4A-D. Thus, an optimum treating region 10 can be formed in accordance with the use of each linear device 1.

The conductive and insulating surfaces 13A-D and 14A-D of the treating region 10 may be formed by photo-litho-etching the front end of a linear device 1A without a treating region 10 of Fig. 2 (A), or they may be formed by masking each surface before forming the immediately outer layer, or they may be formed by any other methods.

If the front end of the linear device 1 is provided with a protector 11, the front end and, therefore, the treating region 10 are prevented from being damaged when the linear device 1 is inserted into a subj ect.

Especially if the protector 11 is given a cone-like, tapering-off shape, the resistance when the linear device 1 is inserted into a subject is reduced. Thus, the damage to the subject and the linear device 1 is reduced.

If the protector 11 is made of an insulating material, electricity does not flow through the front end of the linear device 1 where current density would otherwise be high when electricity is allowed to flow through the conductive layers 3B-D; accordingly, linear devices 1 do not vary in sensitivity and precision if the shapes of their front ends vary due to manufacturing errors.

A connection 20 of substantially the same construction as the treating region 10 may be formed at the rear end of the linear device 1 as shown in Fig. 1. In this case, the areas of connecting surfaces 23A-D and insulating surfaces 24A-D can be made large as compared with the thicknesses of the conductive and insulating layers 3B-D and 4A-D; accordingly, the conductive layers 3B-D can easily be connected to a power supply or the like and short circuits between the conductive layers 3B-D can be prevented without fail.

Although the linear device 1 of Figs. 1 and 2 has an axial core 2 of which the cross section is circular, the cross section may be rectangular or triangular or in any other shapes.

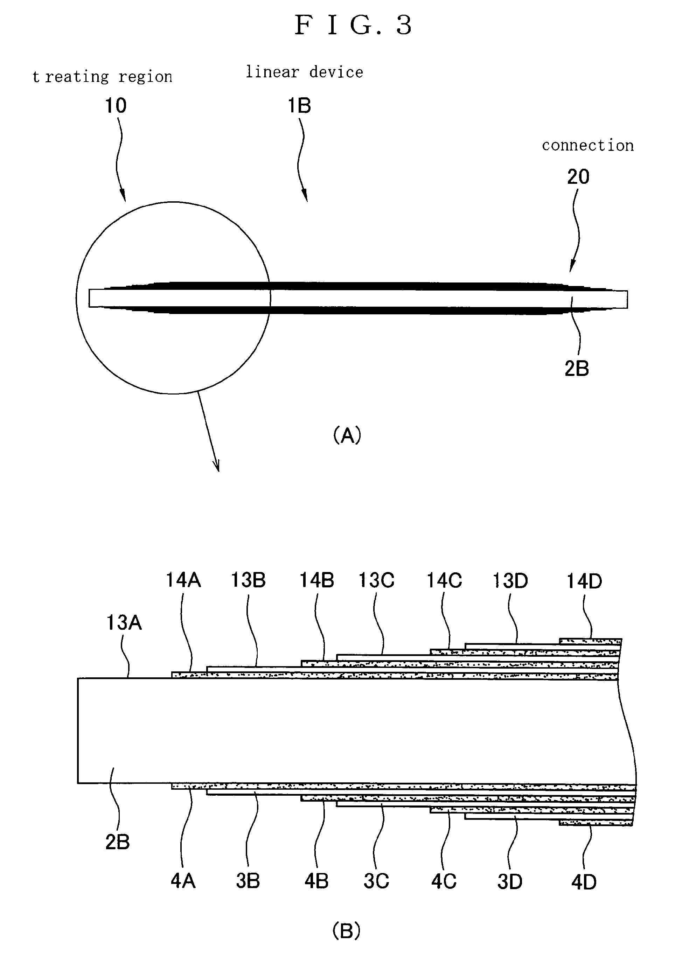

Fig. 3 shows another embodiment 1B of linear device in accordance with the present invention. The linear device 1B has a thin, long plate 2B instead of the axial core 2. Conductive layers 3B-D and insulating layers 4A-D are formed on the plate 2B.

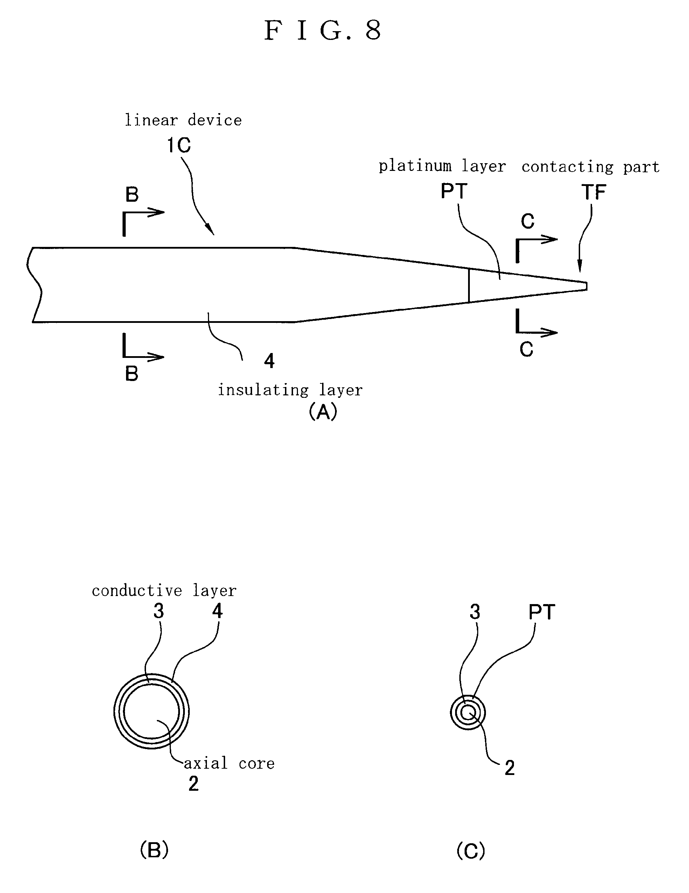

Fig. 8 shows another embodiment 1C of linear device in accordance with the present invention. The linear device 1C comprises an axial core 2, a conductive layer 3, and an insulating layer 4. The conductive layer 3 is formed on the axial core 2; the insulating layer 4, on the conductive layer 3. The conductive layer 3 is exposed at the front end of the linear device 1C to constitute a contacting part "TF". If two linear devices 1C are arranged side by side in a substance and voltage is applied between their conductive layers 3, electricity flows from the contacting part "TF" of one of the two linear devices 1C to that of the other linear device 1C through the substance. Thus, an electric stimulus is given to the substance. The intensity of the current between the contacting parts "TF" of two linear devices 1C arranged side by side varies depending on the properties of substances between the contacting parts "TF"; accordingly, the kinds, densities, etc. of the substances can be determined and measured.

Because the front end of the linear device 1 is tapered off, the resistance when the linear device 1 is inserted into an organism or the like is small. Because of the taper, the contacting part "TF" of the linear device 1 is small; accordingly, electric stimuli can be applied to minute parts and substances in the minute parts can be detected and measured. Because the contact surface between the contacting part "TF" and a subject can be adjusted by changing the size of the contacting part "TF", the extent of a region to which electric stimuli are given and whose properties are measured can be adjusted.

If a platinum layer "PT" is formed on the contacting part "TF", platinum being a chemically stable substance, as shown in Fig. 8, the conductive layer 3 is prevented from coming into direct contact with a subject such as an organism and, hence, from affecting the subject directly; therefore, the conductive layer 3 can be made of various materials.

Instead of the above platinum layer "PT", a layer of any other substance, such as gold or titanium, which does not affect organisms, etc. and is chemically stable may be formed on the contacting part "TF".

The conductive layer 3 may be made of platinum. In this case, if a layer of another substance is formed, as an underlying layer, on the axial core 2 and a conductive layer 3 of platinum is formed on the underlying layer, the platinum sticks fast to the axial core 2.

If the linear device 1 is to be embedded in an organism, it is desirable to cover the linear device 1 except its front end and treating region 10 with thin film of fluororesin, polyurethane, polysiloxane, silicone resin, a polymer similar to phospholipid, or the like which organisms do not reject. Thus, the linear device 1 is prevented from being rejected by an organism wherein it is embedded.

If the above thin film is as thin as one micrometer or porous, the linear device 1 including its front end and treating region 10 can be covered with the thin film. In this case, the thin film does not serve as an insulation layer, but keeps protein and the like, which would disturb the insulation and the like of conductive layers 3B-D if they stuck to the conductive layers 3B-D, from sticking to the conductive layers 3B-D.

Next, how to manufacture the linear device 1 will be described below.

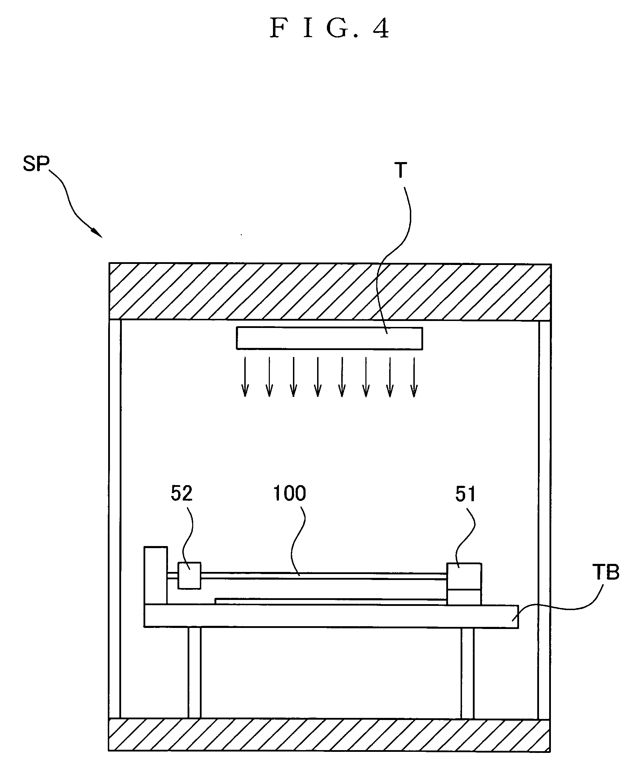

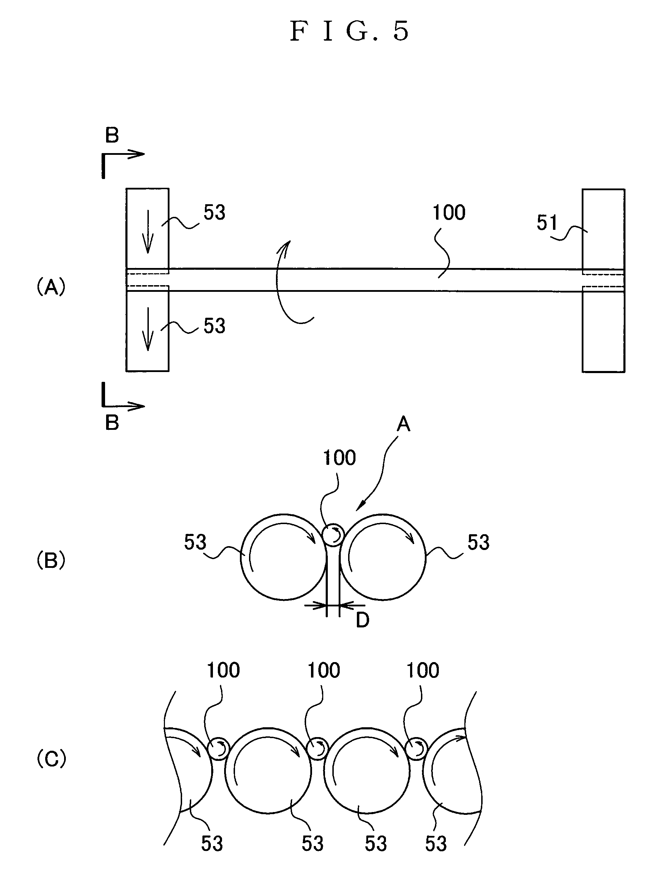

Fig. 4 is a schematic illustration of a device to form layers on a rod 100 which later becomes an axial core 2. Fig. 5 (A) is an illustration of another mechanism for rotating a rod 100 and Fig. 5 (B) is an end view taken along the arrowed line B-B of Fig. 5 (A). Fig. 5 (C) is an illustration of a mechanism for rotating a plurality of rods 100.

In Fig. 4, the reference sign of "SP" is the vacuum chamber of a sputtering device; "T", a target. The reference sign of "TB" is a table on which a rod 100 is placed.

Connected to the vacuum chamber "SP" is a vacuum pump and so on (not shown).

In Figs. 4 and 5, the reference numeral 100 is a rod which becomes an axial core 2 later. In Fig. 4, one end of the rod 100 is held by a holding mechanism 52 such as a known chuck with three jaws. Provided at the back of the holding mechanism 52 is a spindle 52a, which is connected to a motor (not shown) through a reduction gear. The other end of the rod 100 is journaled in a bearing 51 on the table "TB".

Accordingly, when the motor (not shown) is switched on, the rod 100 is rotated by the motor through the spindle 52a and the holding mechanism 52.

Accordingly, the target material can be deposited on the surface of the rod 100 by causing the target "T" to give out a target material onto the surface of the rod 100 while the rod 100 is rotated. Thus, the target material is deposited continuously onto the surface of the rod 100; therefore, a continuous layer of the target material is formed on the rod 100.

After forming a layer of target "T" of a desired thickness, the target "T" is replaced by another target "T" of a different target material to form another layer on the rod 100. Thus, just by changing the targets "T", a plurality of layers can be formed on the rod 100.

Instead of the above mechanism for rotating a rod 100 of Fig. 4, any other mechanisms for rotating a rod or rods 100 may be adopted.

As mentioned earlier, Figs. 5 (A) and (B) show another mechanism for rotating a rod 100. The reference numeral 53 is a pair of rollers. The rollers 53 are so disposed that they are parallel to each other and the gap between them is smaller than the diameter of a rod 100. Accordingly, when one end of a rod 100 is put on and between the rollers 53, or in a space "A" between the rollers 53 as shown in Fig. 5 (B), and the rollers 53 are rotated in one and the same direction, the rod 100 is rotated in the opposite direction. The rollers 53, a bearing 51, and a rod 100 are so disposed that the axis of the rod 100 will be parallel to the axes of the rollers 53. It is desirable to provide a mechanism for holding the rod 100 so that it will not move in its axial directions.

As mentioned earlier, Fig. 5 (C) shows a mechanism for rotating a plurality of rods 100, of which the operating efficiency is high.

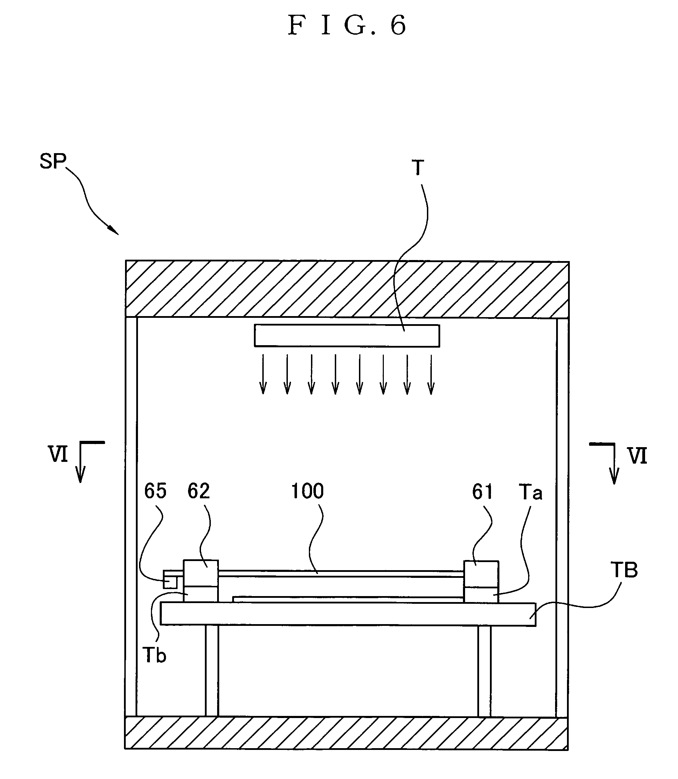

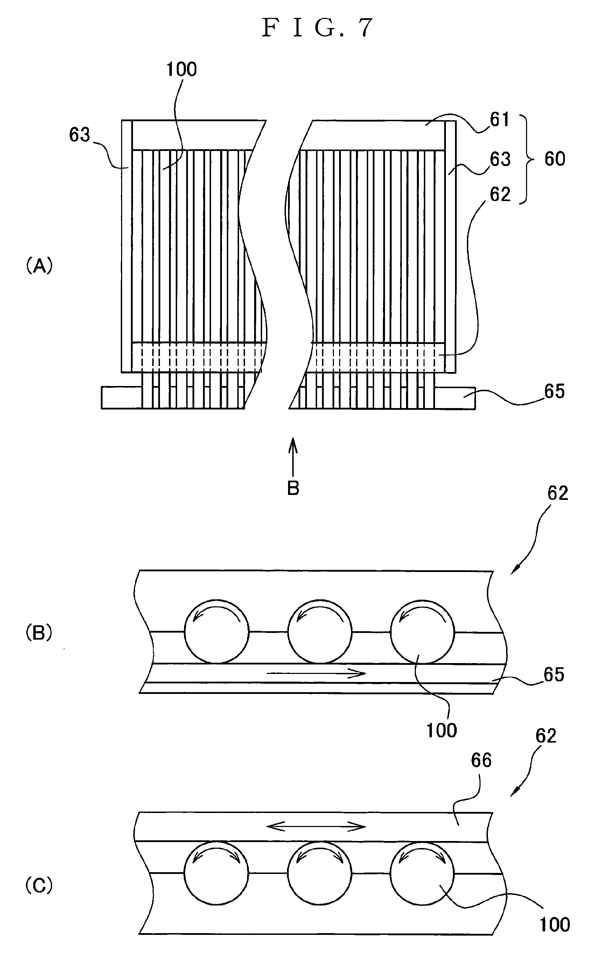

As shown in Figs. 6 and 7, a plurality of rods 100 may be held by a holder 60 and layers may be formed on the rods 100.

The holder 60 comprises end holders 61 and 62, which hold the ends of a plurality of rods 100.

One end of each rod 100 is so journaled in the end holder 61 that said rod 100 will not move in its axial directions.

The other end of said rod 100 is journaled in the end holder 62. More specifically, part near the other end of said rod 100 is so journaled in the end holder 62 that the end will protrude out of the holder 62 for the reason described below.

Connectors 63 are provided between the end holders 61 and 62.

In Fig. 6, the reference sign "TB" is a table and reference signs "Ta" and "Tb" are supports which are set on the table "TB" and support the end holders 61 and 62, respectively.

A conveyor 65 with an endless belt is provided under the ends of the rods 100 protruding out of the end holder 62. The conveyor 65 is so disposed that the top surface of the endless belt will be in contact with the ends of the rods 100 protruding out of the end holder 62.

Accordingly, when the conveyor 65 is switched on, the rods 100 rotate in the direction opposite to the running direction of the endless belt (Fig. 7). Therefore, the target material can be deposited on the surfaces of the rods 100 by causing the target "T" to give out a target material onto the surfaces of the rods 100 while the rods 100 are rotated.

Besides, a plurality of rods 100 can be set in the holder 60 and processed; accordingly, rods 100 can be handled easily and prevented from being damaged or lost.

In addition to the above conveyor 65, any other methods of rotating rods 100 may be adopted. For example, as shown in Fig. 7 (C), a plate-like member 66 may be provided on the ends of the rods 100 protruding out of the end holder 62. In this case, the rods 100 can be rotated clockwise and counterclockwise by moving the member 66 right and left.

The linear device 1 of the present invention will be described more concretely below.

The axial core 2 is made of a superelastic alloy (Ni-Ti alloy) and its diameter is 0.08 mm or less. Three conductive layers 3 of platinum and three insulating layers 4 of polyimide are formed alternately by sputtering and electro-deposition, respectively; thus, a linear device 1 with three conductive layers 3 and three insulating layers 4 formed alternately on the axial core 2 is made.

A desired length of the front end of the linear device 1 is dipped into chloroform and aqua regia alternately. Chloroform and aqua regia dissolve polyimide and platinum, respectively; accordingly, a treating region 10 with three conductive surfaces 13 is formed at the front end of the linear device 1.

Then, one of the three conductive surfaces 13 is plated with silver by anodization and oxidized in a solution of hydrochloric acid by electrolytic oxidation; thus, a reference electrode plated with silver chloride is made.

Another conductive surface 13 is dipped into phosphate buffer (pH 7.4) containing a derivative from pyrrole, glucose oxidase (GOD), and lithium perchlorate for sufficient deaeration and undergoes controlled-potential-electrolysis polymerization at 1.2 V (vs. Ag/AgCl) below the freezing point; thus, a measuring electrode is made.

The remaining conductive surface 13 undergoes no processing to be a counter electrode.

The front end of the linear device 1 is covered with a protector 11 of silicone rubber, and the linear device 1 is entirely coated with polyurethane.

The linear device 1 was embedded in between the scapulae of a rat and the conductive layers 3 were connected to an electrochemical analyzer. Then, a voltage of 1.2 V (vs. Ag/AgCl) was applied to the measuring electrode to detect an electric current corresponding to the density of glucose in the rat.

### Industrial Applicability

The linear device of the present invention can be applied to (i) a device which is applied to an organism or the like and gives an electric, thermal, or chemical stimulus to the organism or the like, (ii) a measuring instrument to measure the constituent elements of substances in or gathered from an organism or the like, and (iii) a device which is applied to an organism or the like and measure electrically, electrochemically, or optically the changes occurring in the organism or the like.

## Claims

1. A linear device which is a linear member comprising:
a base layer extending in the axial directions of the linear member; and
a plurality of layers formed on the base layer and extending in the axial directions of the linear member,
one of said plurality of layers being a conductive layer and one of said plurality of layers being an insulating layer.

2. The linear device according to claim 1, wherein the front end of the linear member is pointed.

3. The linear device according to claim 1 or 2, wherein:
the conductive layer is formed on one side of the base layer;
the insulating layer is formed so as to cover the surface of the conductive layer; and
the conductive layer is exposed at the front end of the linear member to constitute a contacting part.

4. The linear device according to claim 3, wherein a platinum layer is formed on the contacting part.

5. The linear device according to claim 1, 2, 3, or 4, wherein two or more of said plurality of layers are conductive layers and two or more of said plurality of layers are insulating layers, each insulating layer being disposed between the conductive layers.

6. The linear device according to claim 1, 2, 3, 4, or 5, wherein one of said plurality of layers is of a superelastic alloy.

7. The linear device according to claim 1, 2, 3, 4, or 5, wherein one of said plurality of layers is of a superelastic resin.

8. The linear device according to claim 1, 2, 3, 4, 5, 6, or 7, wherein one of said plurality of layers is of a shape-memory material.

9. The linear device according to claim 1, 2, 3, 4, 5, 6, 7, or 8, wherein the width of the linear member is 1-200 µm.

10. The linear device according to claim 1, 2, 3, 4, 5, 6, 7, 8, or 9, wherein the linear member has an axial core serving as the base layer.

11. The linear device according to claim 1, 2, 3, 4, 5, 6, 7, 8, 9, or 10, wherein a detecting agent, which reacts on a certain substance to produce another one, is applied to the surface of one of the conductive layers at the front end of the linear member.

12. The linear device according to claim 1, 2, 3, 4, 5, 6, 7, 8, 9, 10, or 11, wherein one side of the front end of the linear member is provided with a treating region which includes conductive surfaces and insulating surfaces arranged alternately in the directions of the longitudinal center axis of the linear member, each conductive surface being part of the outer surface of one of the conductive layers and each insulating surface being part of the outer surface of one of the insulating layers.

13. The linear device according to claim 12, wherein the front end of the linear member is provided with a protector of an insulating material to cover the front end.
